(19) Europäisches Patentamt
European Patent Office
Office européen des brevets

(11) **EP 2 277 041 B1**

(12) **EUROPÄISCHE PATENTSCHRIFT**

(45) Veröffentlichungstag und Bekanntmachung des Hinweises auf die Patenterteilung:
**19.10.2011 Patentblatt 2011/42**

(21) Anmeldenummer: **09749577.4**

(22) Anmeldetag: **13.05.2009**

(51) Int Cl.:
*G01N 33/00* (2006.01)    *G01N 1/24* (2006.01)

(86) Internationale Anmeldenummer:
**PCT/EP2009/003414**

(87) Internationale Veröffentlichungsnummer:
**WO 2009/141087 (26.11.2009 Gazette 2009/48)**

(54) **VORRICHTUNG UND VERFAHREN ZUM ERFASSEN VON SPURENGASEN**

DEVICE AND METHOD FOR DETECTING TRACE GASES

DISPOSITIF ET PROCÉDÉ DE DÉTECTION DE GAZ À L'ÉTAT DE TRACE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **23.05.2008 DE 102008024769**

(43) Veröffentlichungstag der Anmeldung:
**26.01.2011 Patentblatt 2011/04**

(73) Patentinhaber: **EADS Deutschland GmbH 85521 Ottobrunn (DE)**

(72) Erfinder:
• **MÜLLER, Gerhard**
  **85567 Grafing (DE)**
• **HELWIG, Andreas**
  **80469 München (DE)**

(74) Vertreter: **Schäfer, Matthias W. Patentanwalt Schwanseestrasse 43 81549 München (DE)**

(56) Entgegenhaltungen:
EP-A2- 1 480 035    US-A1- 2007 151 319
US-B1- 6 432 721

**Beschreibung**

Die Erfindung betrifft eine Vorrichtung zum Erfassen von Spurengasen nach dem Oberbegriff des Patentanspruchs 1 und ein Verfahren nach Patentanspruch 8.

Zur Detektion von Gasen und Dämpfen können unterschiedliche Sensoren und Sensorsysteme angewandt werden. Bekannte Gassensoren arbeiten dabei entweder im diffusionslimitierten Betrieb oder werden konstant mit Luft angeströmt. Weit verbreitet sind beheizte Halbleitergassensoren, wie beispielsweise Metalloxide, gassensitive Feldeffekt-Transistoren, etc., als auch auf thermischen Nachweisprinzipien beruhende Sensoren, wie beispielsweise Wärmetönungs- und Wärmeleitfähigkeitssensoren. Wegen der beschränkten Selektivität solcher Sensoren werden sehr oft auch Arrays aus mehreren unterschiedlich querempfindlichen Sensoren oder auch Temperaturmodulationsverfahren angewandt.

In aller Regel erfolgt der Transport der nachzuweisenden Gase zu den sensitiven Oberflächen durch Gasdiffusion. Bedingt durch die dabei auftretenden. Transportverzögerungen als auch durch die Kinetik der Adsorptions- und Desorptionsvorgänge an den sensitiven Oberflächen werden üblicherweise lange Ansprech- und Abklingzeitkonstanten des Sensorsignals beobachtet. Ein deutlich schnelleres Ansprechverhalten kann beobachtet werden, wenn man denselben Sensor aktiv mit dem nachzuweisenden Gas anströmt.

Bezüglich des Spektrums potentiell nachzuweisender Gase ist zu bemerken, dass alle vorgenannten Hochtemperaturgassensoren ein breites Querempfindlichkeitsspektrum aufweisen. Metalloxidsensoren sprechen beispielsweise im Prinzip auf alle Arten von brennbaren Gasen (Kohlenwasserstoffe, $H_2$, CO) sowie auf stark oxidierende Gase ($O_3$, $NO_2$, $SO_2$) an.

Während mit den bekannten Sensoren bei hoher Temperatur alle Gase mit hoher Empfindlichkeit nachgewiesen werden, werden im Bereich niedriger Sensorbetriebstemperaturen, d.h. < 200°C, nur solche Gase nachgewiesen, die leicht in Wasser dissoziieren, beispielsweise $NO_2$ oder $NH_3$. Der Grund für dieses veränderte Nachweisverhalten ist das Vorliegen einer dünnen Wasserschicht auf der sensitiven Halbleiteroberfläche mit einer Dicke d $\sim$ 1-10 nm. Der bei $NO_2$ und bei $NH_3$-Exposition in verschiedene Richtung zeigende Sensor antwort legt nahe, dass der Metalloxidsensor einfach den effektiven pH-Wert in dem Oberflächenwasser anzeigt.

Die US 6 432 721 B1 offenbart beispielsweise ein Gasdetektionssystem mit einer Membranepumpe, einer Pufferkammer, einem mit chemischen Rezeptoren ausgestatteten Sensorkopf und einem Ansaugkanal, wobei die Pumpe eine Kammer aufweist, in welche Gase durch einem ersten und einem zweitem Kanal eingelassen werden können. Dabei wird erst Probenluft eingesaugt und danach mit sauberer Luft gespült.

Vergleicht man bei einer solchen Vergleichsmessung die Zimmertemperatur- mit den Hochtemperaturdaten, dann wird deutlich, dass das Ansprechzeitverhalten bei Zimmertemperatur (RT) und bei Hochtemperatur (410°C) zwar vergleichbar gut, das Abklingzeitverhalten bei Zimmertemperatur (RT) jedoch sehr viel schlechter als bei 410°C ist. Dieses schlechte Abklingverhalten ist bedingt durch die elektrolytische Dissoziation von $NO_2$ in dem Oberflächenwasser und der nachfolgenden Hydratisierung der entstandenen NO3 Ionen:

$$NO_2 + H_2O \rightarrow NO3- + H_3O+ \rightarrow (NO_3^+)aq + (H_3O^+)aq.$$

Durch die stark erhöhte Molekülmasse der hydratisierten Ionen ist das Verdunsten nach Beendigung der $NO_2$ Exposition behindert; eine effektive Wiederherstellung des Sensorbasiswiderstands wird erst nach Beaufschlagung des Sensors mit $NH_3$ beobachtet. In diesem Falle erfolgt keine Verdunstung des gelösten $NO_2$ sondern seine elektrolytische Neutralisation. Ähnliche Gassensierungseffekte können auch an weiteren Halbleitermaterialien beobachtet werden.

Ein Nachteil aller vorgenannten Sensorsysteme ist deren relativ geringe Empfindlichkeit und insbesondere deren relativ schlechtes Ansprech- und Abklingverhalten. Bedingt durch das besonders langsame Abklingen kann es bei Überlagerung mehrerer zeitlich begrenzter Gasexpositionen zu Akkumulations- und Drifteffekten kommen, die die erhaltenen Sensorsignale schwer interpretierbar machen.

Der Erfindung liegt daher die Aufgabe zugrunde, die Nachteile der bekannten Lösungen des Standes der Technik zu vermeiden und eine verbesserte und kostengünstigere Lösung zum Nachweis von Spurengasen zur Verfügung zu stellen.

Diese Aufgabe wird erfindungsgemäß durch eine Vorrichtung zum Erfassen von Spurengasen mit den Merkmalen des Patentanspruchs 1 und ein entsprechendes Verfahren mit den Merkmalen des Patentanspruchs 8 gelöst. Vorteilhafte Ausgestaltungen und Weiterbildungen der Erfindung sind in den abhängigen Ansprüchen angegeben.

Das erfindungsgemäße Gasdetektionssystem zum Erfassen von Gasen, Dämpfen und biologischen Pathogenen ist dabei mit zumindest einem Rezeptor 4 vorgesehen, der an einer Leitung angeordnet ist, welche Umgebungsluft mit einem Luftspeicher verbindet, wobei das Gasdetektionssystem als "atmendes" System ausgebildet ist, welches im Luftspeicher saubere $CO_2$ tragende mit feuchte gesättigte Luft aufweist, wobei die Arbeitstemperatur des Gasdetektionssystems Raumtemperatur ist. Das Aufbringen von Messproben erfolgt dabei in Form eines vom offenen Ende der Leitung angesaugten kalten trockenen Luftvolumens (Einatmen) aus der Umgebungsluft. Dieses Strömt durch die Leitung und gelangt dadurch auf einen Rezeptor. Durch das. Anlagern von entsprechender Gase, Dämpfe oder biologischer Pathogene an den Rezeptor, ändert sich der an den Rezeptor angelegte Strom. Je nach Art des Stoffes und des jeweiligen Rezeptors können hier unterschiedliche Messergebnisse auftreten. Anschließend erfolgt das Spülen der Oberfläche des Rezeptors 4 (der so genannte Ausatemvorgang) durch Ausstoßen eines warmen feuchten Luftvolumens aus dem Luftspeicher nach dem Messschritt.

Hierdurch werden die Nachteile der bekannten Lösungen des Standes der Technik vermieden und eine verbesserte und kostengünstigere Lösung zum Nachweis von Spurengasen zur Verfügung gestellt.

Eine vorteilhafte Ausführungsform der Erfindung sieht vor, dass der zumindest ein Rezeptor mit einer Feuchteschicht zum Dissoziieren von löslichen Stoffen vorgesehen ist. Dies ermöglicht die Erfassung von in Flüssigkeit lösbaren Stoffen.

Eine weitere vorteilhafte Ausführungsform der Erfindung sieht vor, dass die Temperatur der im Luftspeicher vorhandenen Luft etwa 37°C beträgt. Diese Temperatur hat sich in Messungen als ausreichend herausgestellt, um die geforderte Messgenauhigkeit zu gewärleisten. Höhere Temperaturen sind ebenfalls möglich, erfordern jedoch einen höheren Energieaufwand.

Noch eine vorteilhafte Ausführungsform der Erfindung sieht vor, dass ein Array unterschiedlicher Rezeptoren vorgesehen ist. Hierdurch können unterschiedliche Gase, Dämpfe und biologische Pathogene gleichzeitig parallel zueinander erfasst werden.

Eine Weiterbildung der oben beschriebenen Erfindung betrifft die Verwendung von Halbleitersubstraten. Auf Halbleitersubstraten adsorbierte Feuchtefilme können mit dem adsorbierten Feuchtefilm Ladungsträger austauschen. Auf diese Art und Weise wird die Oberflächenleitfähigkeit und die Raumladekapazität unter der Halbleiteroberfläche moduliert. Arbeiten an Metalloxiden und Diamantschichten haben gezeigt, dass hierdurch der effektive pH-Wert in der Feuchteschicht vermessen werden kann.

Zur stoffspezifischen Detektion kann das Substrat (IDK / Interdigitalkontakt, Halbleiter) mit speziell bindenden molekularen Rezeptoren beschichtet werden. Eine vorteilhafte Ausführungsform betrifft die Beschichtung des Substrats mit geruchsbindenden Proteinen.

Eine weitere vorteilhafte Ausführungsform betrifft das Aufspüren von Bakterien. Hierbei sind bestimmte Bakterien als Rezeptoren 4 vorgesehen, die sich mit geeigneten Antikörpern (Proteinen) verbinden. Der entstehende Bakterien-Antikörper-Komplex kann dann mit geeigneten Methoden detektiert werden. Auch hier kann das Echtzeit-Sampling-Verfahren angewandt werden.

Das erfindungsgemäße Verfahren zum Erfassen von Gasen, Dämpfen und biologischen Pathogenen mit dem oben beschriebenen Gasdetektionssystem weist folgende Schritte auf:

- Aufbringen von Messproben in Form eines angesaugten kalten trockenen Luftvolumens (Einatmen) aus der Umgebungsluft auf einen Rezeptor; und

- Spülen (Ausatmen) der Oberfläche des Rezeptors 4 durch Ausstoßen eines warmen feuchten Luftvolumens aus einem Luftspeicher nach dem Messschritt.

Eine vorteilhafte Weiterbildung des erfindungsgemäßen Verfahrens sieht vor, dass vor dem Aufbringen der Messproben ein Spülen (Ausatmen) der Oberfläche des Rezeptors durch Ausstoßen eines warmen feuchten Luftvolumens aus dem Luftspeicher erfolgt.

Eine weitere vorteilhafte Weiterbildung des erfindungsgemäßen Verfahrens sieht vor, dass die Messung durch eine rechnergestützte Auswertungseinheit in einem Echtzeit-Sampling-Verfahren bei Raumtemperatur erfolgt.

Weitere die Erfindung verbessernde Maßnahmen werden nachstehend gemeinsam mit der Beschreibung eines bevorzugten Ausführungsbeispiels der Erfindung anhand der Figuren näher dargestellt. Es zeigen:

Fig. 1    ein schematisches Funktionsprinzip eines erfindungsgemäßen "atmenden" Gasdetektionssystems;

Fig. 2    eine Messung von $NH_3$ im Atemmodus mit Hilfe einer Pt-IDK (Platin-Interdigitalkontakt) Struktur,

Fig. 3    einen Ausschnitt aus Fig. 2 der die Messung von $NH_3$ im Atemmodus mit Hilfe eines Pt-IDK zeigt;

Fig. 4    einen Ausschnitt aus Fig. 2, der die Messung von $NH_3$ im Atemmodusmit Hilfe eines Pt-IDK zeigt.

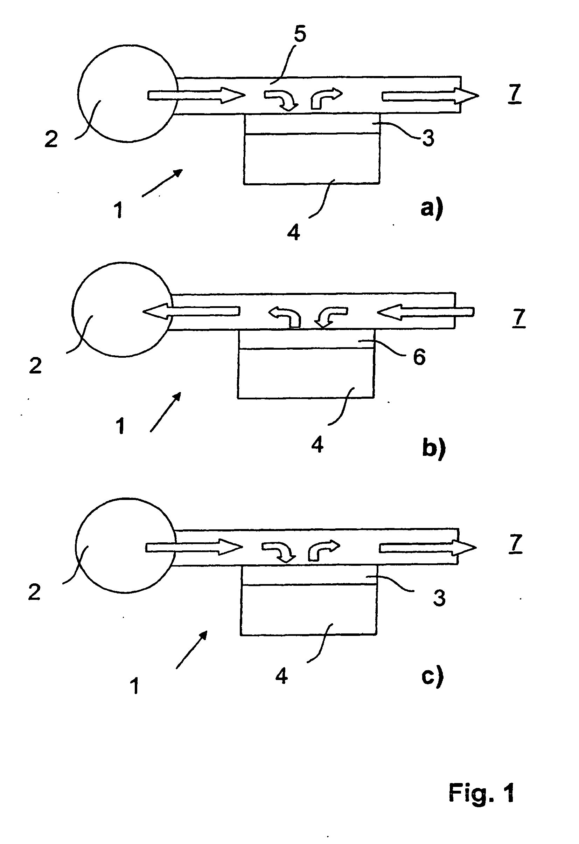

Das erfindungsgemäße Messverfahren ist schematisch in Figur 1 dargestellt. Hier wird ein "atmendes" Gasdetektionssystem 1 mit Messungen an Pt-IDK-Rezeptoren gezeigt, d.h. im Sensorbetrieb wird ständig zwischen warmer feuchter und kalter trockener Luft gewechselt, was nachfolgend als Atemmodus bezeichnet wird. Hierdurch wird ein Echtzeit-Sampling-Verfahren zum Erfassen von Gasen, Dämpfen und biologischen Pathogenen bei Raumtemperatur (~ 20°C) geschaffen. Durch den Atemmodus des Gasdetektionssystem 1 kommt es zum ständigen Wechsel zwischen Säuberung der an der Leitung 5 angeordneten Rezeptoren 4 und Analyse der Umgebungsluft 7. Die Leitung 5 verbindet einen Luftspeicher 2 mit der Umgebungsluft 7. Dazwischen sind die Rezeptoren 4 angeordnet. Die Messfrequenz beträgt einen Atemzyklus. Atmet das Gasdetektionssystem 1 durch die Leitung 5 aus, wie in Figur 1a) gezeigt, gibt ein am Ende der Leitung 5 angeordneter Luftspeicher 2 saubere $CO_2$ tragende Luft frei. Dieses Luftvolumen ist mit Feuchte gesättigt und hat eine Temperatur von ca. 37°C. Die Luftströmungsrichtung in der Leitung 5 ist durch die Pfeilrichtung angedeutet. Da das Gasdetektionssystem 1 bei Raumtemperatur arbeitet, also deutlich kühler ist, kondensiert die Feuchte auf den Oberflächen der Rezeptoren 4 in dem Gasdetektionssystem 1 (BET - Isotherme) und bildet dort einen sauberen Feuchtefilm 3 von mehreren Moleküllagen. Der Zustand der Rezeptoren 4 ist dadurch eindeutig definiert.

Während des Einatmens, wie in Figur 1b) gezeigt, kommt es einerseits zu einem Dissoziieren von löslichen Stoffen in der Feuchteschicht 6 und andererseits zum Andocken von Molekülen auf geeigneten Rezeptoren 4. Da während eines Atmenzugs ein Luftvolumen von der Größenordnung 1 Liter an der dünnen Feuchteschicht vorbeigeführt wird, wie durch die Pfeile angedeutet, kann es im Fall einer erfolgreichen Dissoziation zu einer beträchtlichen Aufkonzentration der eingeatmeten Analytgase kommen. Betrachtet man z.B. eine Oberfläche von $1cm^2$ Fläche und eine darauf adsorbierte Feuchteschicht von 10nm Dicke, so kann maximal eine Aufkonzentration K von

$$K = V_{Luft}/V_{Feuchteschicht} \sim 1000cm^3/10^{-6}cm^3 \sim 10^9$$

erreicht werden. Weiterhin ist zu berücksichtigen, dass bedingt durch die geringere Temperatur und Luftfeuchte der eingeatmeten Umgebungsluft 7, die Feuchteschicht 6 auf den Oberflächen der Rezeptoren 4 verdunstet. Dies begrenzt die Aufnahmefähigkeit der Feuchteschicht 6 und zwingt die dissoziierten und hydratisierten Analytmoleküle schließlich zum Andocken an die spezifischen Rezeptoren 4. Eine optimale Detektion von unterschiedlichsten Verunreinigungen ist jetzt möglich.

Beim darauf folgenden Spülen (Ausatmen), wie in Figur 1c) gezeigt, erfolgt wieder eine Reinigung der Rezeptoren 4 und eine Neubelegung mit einer sauberen Feuchteschicht 3.

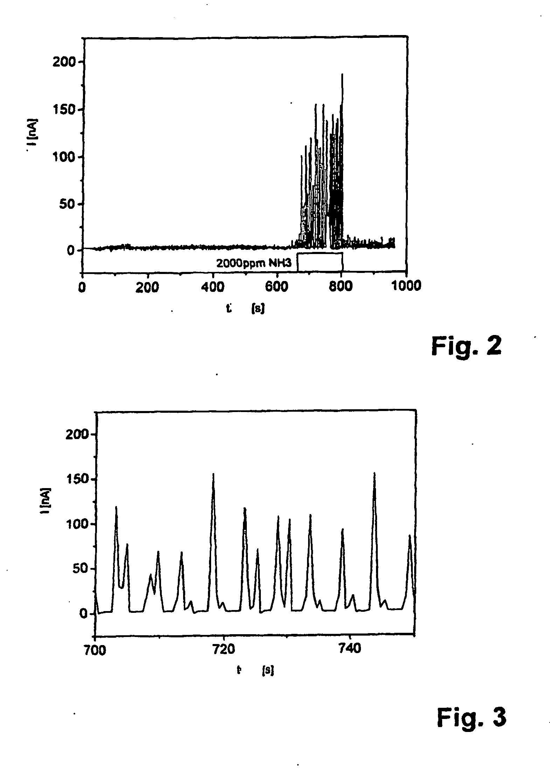

Fig. 2 zeigt als Beispiel die Ergebnisse einer $NH_3$ Messung im Atemmodus mit einfachen Platin-Interdigitalelektroden. In dem Diagramm ist der Strom 1 in nA über der Zeit t in Sekunden aufgetragen.

Während des Ausatmens wird warme, feuchte Luft auf dem kälteren IDK kondensiert. Die kondensierte Feuchte bildet einen leitfähigen Film auf der Oberfläche. Der Strom beträgt jetzt wenige nA. Beim nachfolgenden Einatemprozess wird trockene Luft über den IDK gesogen; die Feuchte verdunstet und die Leitfähigkeit verschwindet. Betrachtet man einen Zeitraum, der sich über viele Ein- und Ausatemzyklen erstreckt (t < 650 s), beobachtet man ein kleines, periodisches und stark verrauschtes Sensorsignal. Gibt man der Umgebungsluft nun eine Konzentration von 200ppm $NH_3$ zu, so löst sich dieses Gas in der Feuchteschicht und erhöht durch basische Dissoziation die Leitfähigkeit des Feuchtefilms. Das Signal steigt auf das ca. 100fache seines Reinluftwerts an.

Fig. 3 zeigt den Zeitbereich unter $NH_3$ Exposition in höherer Auflösung. Hier ist deutlich zu erkennen, dass während der Spülphase (Ausatmen) das Signal wieder komplett auf Null zurückgeht.

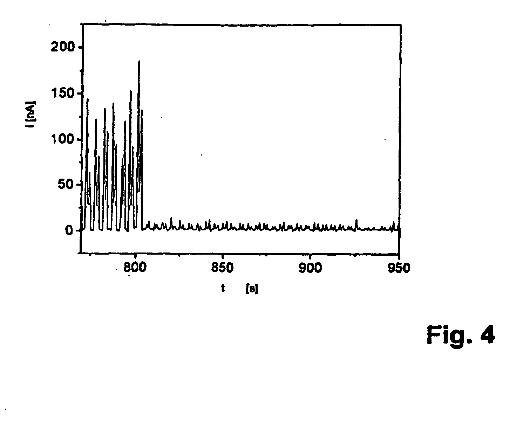

In Fig. 4 wird ein weiterer Ausschnitt aus Fig. 2 dargestellt. Hier ist deutlich zu erkennen, dass sofort nach dem Beenden der Gasexposition (800s) das Messsignal auf seinen Anfangswert zurückfällt. Eine langandauernde Desorptionsphase des Gases, wie bei normalem, diffuserem Sensorbetrieb, kann somit nicht beobachtet werden.

**Patentansprüche**

1. Als "atmendes" System ausgebildetes Gasdetektionssystem (1) zum Erfassen von Gasen, Dämpfen und biologischen Pathogenen, wobei zumindest ein Rezeptor (4) vorgesehen ist, der an einer Leitung (5) angeordnet ist, **dadurch gekennzeichnet, dass** die Leitung (5) die Umgebungsluft (7) mit einem Luftspeicher (2) verbindet, welcher saubere, $CO_2$ tragende, mit Feuchte gesättigte Luft aufweist, wobei die Arbeitstemperatur des Gasdetektionssystems Raumtemperatur ist.

2. Gasdetektionssystem nach Anspruch 1, wobei der zumindest eine Rezeptor (4) mit einer Feuchteschicht (3, 6) zum Dissoziieren von löslichen Stoffen vorgesehen ist.

3. Gasdetektionssystem nach einem der vorgenannten Ansprüche, wobei die Temperatur der im Luftspeicher vorhandenen Luft etwa 37°C beträgt.

4. Gasdetektionssystem nach einem der vorgenannten Ansprüche, wobei ein Array unterschiedlicher Rezeptoren vorgesehen ist.

5.  Gasdetektionssystem nach einem der vorgenannten Ansprüche, wobei als Rezeptor (4) zumindest ein Pt-IDK, ein Halbleitersubstrat, eine Metalloxidschicht und/oder eine Diamantschicht vorgeshen ist.

6.  Gasdetektionssystem nach einem der vorgenannten Ansprüche, wobei speziell bindenden molekulare Rezeptoren (4), insbesondere geruchsbindenden Proteinen, vorgesehen sind.

7.  Gasdetektionssystem nach einem der vorgenannten Ansprüche, wobei bestimmte Bakterien als Rezeptoren (4) vorgesehen sind, die sich mit geeigneten Antikörpern (Proteinen) verbinden.

8.  Verfahren zum Erfassen von Gasen, Dämpfen und biologischen Pathogenen mit einem Gasdetektionssystem nach Patentanspruch 1, wobei das Verfahren folgende Schritte aufweist:

    - Aufbringen von Messproben in Form eines angesaugten kalten trockenen Luftvolumens (Einatmen) aus der Umgebungsluft (7) auf einen Rezeptor (4); und
    - Spülen (Ausatmen) der Oberfläche des Rezeptors 4 durch Ausstoßen eines warmen feuchten Luftvolumens aus einem Luftspeicher (2) nach dem Messschritt.

9.  Verfahren nach Patentanspruch 8, wobei vor dem Aufbringen der Messproben ein Spülen (Ausatmen) der Oberfläche des Rezeprors 4 durch Ausstoßen eines warmen feuchten Luftvolumens aus dem Luftspeicher (2) erfolgt.

10. Verfahren nach Patentanspruch 8 oder 9, wobei die Messung durch eine rechnergestützte Auswertungseinheit in einem Echtzeit-Sampling-Verfahren bei Raumtemperatur erfolgt.

## Claims

1.  A gas detection system (1), designed as a "breathing" system, for detecting gases, vapours and biological pathogens, wherein at least one receptor (4) is provided which is arranged in a conduit (5), **characterised in that** the conduit (5) connects the ambient air (7) with an air storage device (2) that comprises clean, $CO_2$-carrying air saturated with moisture, wherein the operating temperature of the gas detection system is room temperature.

2.  The gas detection system according to claim 1, wherein the at least one receptor (4) is provided with a humidity layer (3, 6) for dissociating soluble substances.

3.  The gas detection system according to one of the preceding claims, wherein the temperature of the air present in the air storage device is approximately 37°C.

4.  The gas detection system according to any one of the preceding claims, wherein an array of different receptors is provided.

5.  The gas detection system according to any one of the preceding claims, wherein at least one Pt-IDC, a semiconductor substrate, a metal oxide layer and/or a diamond layer is provided as a receptor (4).

6.  The gas detection system according to any one of the preceding claims, wherein specially binding molecular receptors (4), in particular odour-binding proteins, are provided.

7.  The gas detection system according to any one of the preceding claims, wherein specific bacteria are provided as receptors (4), which combine with suitable antibodies (proteins).

8.  A method for acquiring gases, vapours and biological pathogens with a gas detection system according to claim 1, wherein the method involves the following steps:

    - applying samples in the form of an aspirated cold dry volume of air (inhaling) from the ambient air (7) to a receptor (4); and
    - rinsing (exhaling) the surface of the receptor (4) by ejecting a warm humid volume of air from an air storage device (2) after the measuring step.

9.  The method according to claim 8, wherein, prior to the application of the samples, rinsing (exhaling) of the surface of the receptor (4) takes place by ejecting a warm humid volume of air from the air storage device (2).

10. The method according to claim 8 or 9, wherein measuring takes place by means of a computer-assisted evaluation unit in a real-time sampling method at room temperature.

**Revendications**

1. Un dispositif de détection de gaz (1) conçu comme un système « respirant » pour la détection de gaz, vapeurs et pathogènes biologiques, présentant au minimum un récepteur (4) placé sur un conduit (5), **caractérisé en ce que** le conduit (5) relie l'air ambiant (7) à un réservoir d'air (2) qui se compose d'air propre, porteur de $CO_2$ et saturé d'humidité ; la température de travail du dispositif de détection de gaz étant la température ambiante.

2. Le dispositif de détection de gaz selon la Revendication 1 où le au moins un récepteur (4) est doté d'une couche humide (3, 6) destinée à dissocier les substances solubles.

3. Le dispositif de détection de gaz selon l'une des Revendications précédentes où la température de l'air présent dans le réservoir d'air est d'environ 37°C.

4. Le dispositif de détection de gaz selon l'une des Revendications précédentes où un réseau de différents récepteurs est prévu.

5. Le dispositif de détection de gaz selon l'une des Revendications précédentes où, en guise de récepteur (4), est prévu au minimum un condensateur interdigital en Pt, un substrat semi-conducteur, une couche d'oxyde métallique et/ou une couche de diamant.

6. Le dispositif de détection de gaz selon l'une des Revendications précédentes où des récepteurs moléculaires liants spécifiques (4), en particulier des protéines liant les odeurs, sont prévus.

7. Le dispositif de détection de gaz selon l'une des Revendications précédentes où des bactéries définies sont prévues en tant que récepteurs (4), qui se lient avec des anticorps adaptés (protéines).

8. Procédé de détection de gaz, vapeurs et pathogènes biologiques avec un dispositif de détection de gaz selon la Revendication 1, où le procédé comprend les étapes suivantes :

   - mise en place d' échantillons de mesure sous forme d'un volume d'air sec et frais aspiré (inspiration) à partir de l'air ambiant (7) sur un récepteur (4) ; et
   - purge (expiration) de la surface du récepteur (4) par l'émission d'un volume d'air chaud et humide à partir du réservoir d'air (2) après le processus de mesure.

9. Le procédé selon la Revendication 8 où une purge (expiration) de la surface du récepteur (4) est réalisée avant la mise en place des échantillons de mesure par l'émission d'un volume d'air chaud et humide à partir du réservoir d'air (2).

10. Le procédé selon la Revendication 8 ou 9 où la mesure est réalisée au moyen d'une unité d'exploitation dans le cadre d'un procédé d'échantillonnage en temps réel à température ambiante.

**Fig. 1**

**Fig. 2**

**Fig. 3**

**Fig. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6432721 B1